# EUROPEAN PATENT APPLICATION

(11) **EP 1 184 034 A2**
(43) Date of publication of application: **06.03.2002**
(21) Application number: 01111018.6
(22) Date of filing: 08.05.2001
(51) Int. Cl.: A61K 31/00

(54) **Oral drug composition containing a verapamil derivative as a drug-absorption promotor**

(30) Priority: 11.08.2000 KR 2000046643
(71) Applicant: Hanmi Pharm. Co., Ltd., Hwaseong-gun, Kyungki-do 445-910 (KR)
(72) Inventor: Woo, Jong-Soo, Cheoncheonjugong Apt. 118-203, Suwon-si, Kyungki-do 440-330 (KR); Yoo, Sung-Eun, Daejeon 305-390 (KR)
(74) Representative: Lorenz, Werner, Dr.-Ing.

(57) **Abstract**

The bioavailability of a drug which is not readily absorbed in the digestive tract can be greatly enhanced by administering an oral composition comprising a drug and a compound of formula (I): 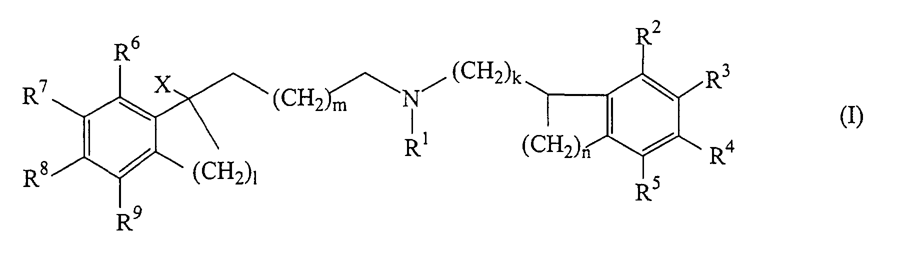 wherein,
X is CN, COOH, COOR¹⁰(wherein R¹⁰ is C₁₋₂ alkyl), SO₂Ph or SPh;
k, l, m and n are each independently an integer of 0 to 4;
R¹ is H or C₁₋₃ alkyl; and
R², R³, R⁴, R⁵, R⁶, R⁷, R⁸ and R⁹ are each independently H, OH or C₁₋₃ alkoxy.

## Description

### FIELD OF THE INVENTION

The present invention relates to an improved oral composition of a drug which by itself is not readily absorbed in the digestive tract, said composition comprising the drug and a verapamil derivative which does not cause any adverse side effects.

### BACKGROUND OF THE INVENTION

Drugs which are not readily absorbed in the digestive tract include many anti-cancer agents, e.g., actinomycin D, doxorubicin, daunomycin, vincristine, vinblastine, colchicine, paclitaxel, docetaxel, etoposide and hydroxyrubicin; immunosuppressive agents, e.g., cyclosporin A and FK-506; and hypotensive agents, e.g., verapamil and nicardipine. These drugs, which are generally administered by injection, are not effective when orally administered due to the inhibitive action of p-glycoprotein present in the intestinal wall(Wacher, V. J. *et al*., *Advanced Drug Delivery Reviews, 20,* p99-112(1996); and Sparreboom, A. *et al*., *Proc*. *Natl*. *Acad*. *Sci., 94*, p2031-2035(1997)).

Paclitaxel, a representative of such drugs, is absorbed only to the extent of 1 % or less when orally administered. Because it is sparingly soluble in water, a typical injection formulation thereof is prepared by employing a 1:1(v/v) mixture of ethanol and Cremophor® EL. However, such an injection formulation must be administered over a long period of time, often with other medication, so as to avoid possible adverse hypersensitive allergic reactions caused by Cremophor® EL.

Accordingly, there have been numerous efforts to develop oral formulation of the above-mentioned drugs by incorporating therein an inhibitor of p-glycoprotein.

For instance, International Patent Publication No. WO98-53811(1998.12.03) discloses a method for enhancing bioavailability of paclitaxel in an oral formulation by co-administering cyclosporin A, a strong immunosuppressive agent. Further, International Patent Publication No. WO97-27855(1998.12.03) teaches a method of using an oral composition of paclitaxel containing cinchonine, another p-glycoprotein inhibitor. However, these methods have the problem of serious adverse effects caused by cyclosporin A and cinchonine.

Accordingly, there exists a demand for an improved oral preparation of paclitaxel or other hardly absorbable drug which is free from above-mentioned problems. The present inventors have endeavored to develop such an oral composition having enhanced drug bioavailability and have found that the absorption of paclitaxel and other drugs in the digestive tract can be greatly facilitated when a verapamil derivative, which by itself has no pharmaceutical activity and causes no adverse side effects, is administered therewith.

### SUMMARY OF THE INVENTION

It is, therefore, an object of the present invention to provide an improved oral composition of a drug which is not readily absorbed in the digestive tract.

### BRIEF DESCRIPTION OF THE DRAWINGS

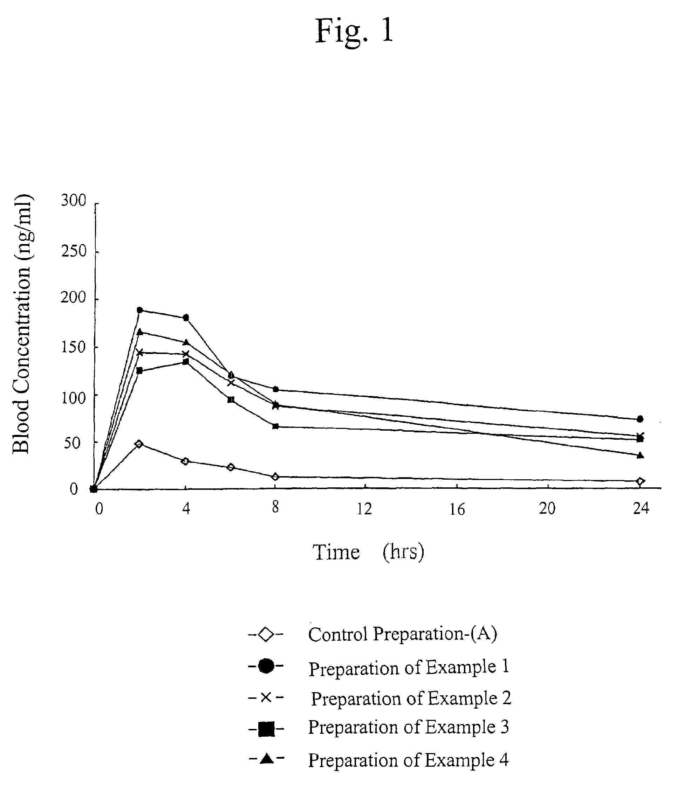

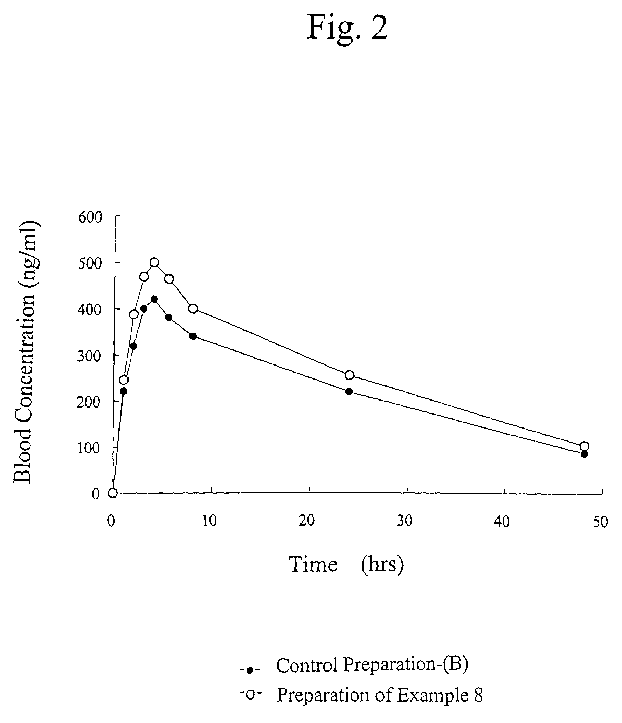

The above and other objects and features of the present invention will become apparent from the following description of the invention, when taken in conjunction with the accompanying drawings, Figs. 1 and 2, which respectively show the bioavailabilities of the inventive oral preparations and control preparations.

### DETAILED DESCRIPTION OF THE INVENTION

In accordance with one aspect of the present invention, there is provided an oral composition comprising a drug which by itself is not readily absorbed in the digestive tract and a compound of formula (I): 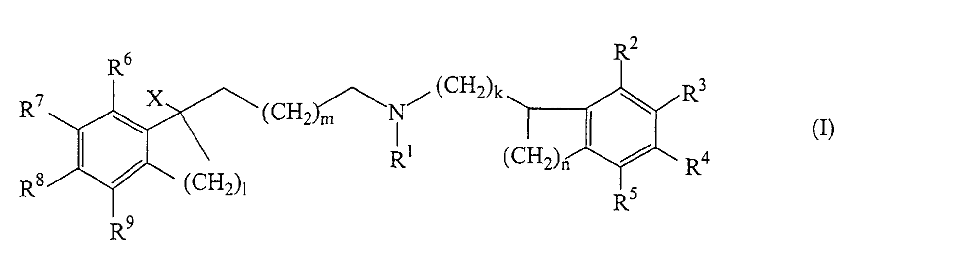 wherein,
X is CN, COOH, COOR¹⁰(wherein R¹⁰ is C₁₋₂ alkyl), SO₂Ph or SPh;
k, 1, m and n are each independently an integer of 0 to 4;
R¹ is H or C₁₋₃ alkyl; and
R², R³, R⁴, R⁵, R⁶, R⁷, R⁸ and R⁹ are each independently H, OH or C₁₋₃ alkoxy.

The inventive oral composition makes it possible to raise the *in vivo* bioavailability of a hardly absorbable drug to a high level without adverse side effects. The inventive composition may further comprise pharmaceutically acceptable carriers for maintaining the drug absorption at a constant rate, examples of said carriers including a co-surfactant, a surfactant and an oil.

The components of the inventive composition are described in detail as follows.

### (1) Pharmacologically active ingredient (drug)

The pharmacologically active ingredient of the inventive composition is any one of those drugs which are not readily absorbed in the digestive tract due to the inhibitive action of p-glycoprotein. Exemplary drugs include anti-cancer agents, e.g., actinomycin D, doxorubicin, daunomycin, vincristine, vinblastine, colchicine, paclitaxel, docetaxel, etoposide and hydroxyrubicin; immunosuppressive agents, e.g., cyclosporin A and FK-506 (Tacrolimus, Fujisawa LTD.); and hypotensive agents, e.g., verapamil and nicardipine.

### (2) Compound of formula (I)

Unlike the conventional p-glycoprotein inhibitors, e.g., cyclosporin A, cinchonine and verapamil, the compound of formula (I) by itself has no pharmacological activity, and consequently causes no side effects, while enhancing the bioavailability of a hardly absorbable drug by inhibiting p-glycoprotein present in the intestinal wall. The compound of formula (I) is disclosed in Korean Patent Publication No. 1999-0030722(1999.5.6) as an agent for negating cancer cell's resistance toward various anti-cancer agents.

Preferred compounds of formula (I) are those wherein X is CN or COOR¹⁰(wherein R¹⁰ is C₁₋₂ alkyl); k, 1, m and n are each independently an integer of 1 to 3; R¹ is methyl; and R², R³, R⁴, R⁵, R⁶, R⁷, R⁸ and R⁹ are each independently H or methoxy.

In particular, 1-(3-{[2-(3,4-dimethoxyphenyl)ethyl]methylamino} propyl)-4,5-dimethoxyindane-1-carbonitrile, 1-(3-{[2-(3,4-dimethoxyphenyl) ethyl]methylamino}propyl)-4,5-dimethoxyindane-1-carboxylic methyl ester, 1-(3-{[2-(3,4-dimethoxyphenyl)ethyl]methylamino}propyl)-5,6-dimethoxy-1,2 ,3,4-tetrahydronaphthalene-1-carbonitrile, and 1-(3-{[2-(2,3,4-trimethoxyphenyl)ethyl]methylamino}propyl)-5,6-dimethoxy-1,2,3,4-tetrahydro naphthalene-1-carbonitrile exhibit superior effects when administered with paclitaxel or cyclosporin A.

The compound of formula (I) may be used in an amount ranging from 0.1 to 50 parts by weight, preferably, 0.5 to 20 parts by weight based on 1 part by weight of the pharmacologically active ingredient.

The inventive oral composition comprising a drug and the compound of formula (I) greatly enhances the bioavailability of the drug regardless how the composition is prepared. However, it is preferred to prepare the composition in the form of an emulsion or micro-emulsion by employing a solvent capable of dissolving the drug and the compound of formula (I). The resulting preparation exhibits a relatively constant absorption rate, when administered to various individuals. For the preparation of such an emulsion or micro-emulsion the following components may be further employed.

### (3) Co-surfactant

Any of the conventional pharmaceutically acceptable amphiphilic(both hydrophilic and hydrophobic) solvents may be employed as a co-surfactant for the inventive composition, so long as it is capable of dissolving both the drug and the compound of formula (I), and forming an emulsion. Thus, the co-surfactant solubilizes the active ingredient, forms a uniform emulsion or micro-emulsion thereof, and keeps the emulsion stable during storage.

Suitable co-surfactants that may be used in the present invention include ethanol, propylene glycol(1,2-dihydroxypropane), polyethylene glycol, e.g., having a molecular weight of 200 to 600, propylene carbonate(4-methyl-2-oxo-1,3-dioxolane), transcutol(diethyleneglycol monoethyl ether), glycofurol(tetrahydrofurfuryl alcohol polyethylene glycol ether), dimethyl isosorbide(1,4:3,6-dianhydro-2,5-dimethyl-D-glucitol) or a mixture thereof, wherein ethanol and dimethyl isosorbide is preferred.

### (4) Surfactant

The surfactant of the inventive composition promotes the formation of a stable emulsion of an oil containing the active ingredients and the co-surfactant in an aqueous medium. Various surfactants inclusive of pharmaceutically acceptable anionic, cationic, non-ionic and amphiphilic surfactants may be used in the present invention.

Representative examples of the surfactant include:
(1) polyoxyethylene glycolated natural or hydrogenated vegetable oils such as polyoxyethylene glycolated natural or hydrogenated castor oil(Cremophor® and HCO® , BASF),
(2) polyoxyethylene-sorbitan-fatty acid esters wherein fatty acid is mono- or tri-lauric, palmitic, stearic or oleic acid(Tween® , ICI),
(3) polyoxyethylene fatty acid esters such as polyoxyethylene stearic acid ester(Myrj® , ICI),
(4) polyoxyethylene-polyoxypropylene copolymer(Pluronic® , BASF),
(5) polyoxyethylene-polyoxypropylene block copolymer(Poloxamer® , BASF),
(6) sodium dioctyl sulfosuccinate or sodium lauryl sulfate,
(7) phospholipids,
(8) propylene glycol mono- or di-fatty acid esters such as propylene glycol dicaprylate, propylene glycol dilaurate, propylene glycol isostearate, propylene glycol laurate, propylene glycol ricinoleate and propylene glycol caprylic-capric acid diester(Miglyol® 840, Huls),
(9) trans-esterification products of natural vegetable oil triglycerides and polyalkylene polyols(Labrafil® M, Gattefosse),
(10) mono-, di- or mono/di-glycerides such as caprylic/capric acid mono- and di-glycerides(Imwitor® , Huls),
(11) sorbitan fatty acid esters such as sorbitan monolauryl, sorbitan monopalmityl and sorbitan monostearyl esters(Span® , ICI), and
(12) sterols or derivatives thereof such as cholesterol, pytosterol and cytosterol.

The surfactant may be selected depending on the kind of the oil component used. Among the above-mentioned surfactants, polyoxyethylene glycolated natural or hydrogenated vegetable oils and polyoxyethylene-sorbitan-fatty acid esters are preferably used in the present invention.

### (5) Oil

The oil component used in the present invention should be compatible with the co-surfactant and surfactant and capable of forming a stable emulsion in an aqueous medium. It may be one of the pharmaceutically acceptable oils capable of dissolving the drug and the compound of formula (I).

Representative examples of the oil include:
(1) fatty acid triglycerides, preferably medium fatty acid triglycerides, such as fractionated coconut oil(Miglyol® 812N, Huls),
(2) mono-, di- or mono/di-glycerides, preferably mono- or di-glycerides of oleic acid,
(3) esters of fatty acids and monovalent alkanols, preferably esters of C₈₋₂₀ fatty acids and C₂₋₃ monovalent alkanols, such as isopropyl myristate, isopropyl palmitate, ethyl linoleate and ethyl oleate,
(4) natural vegetable or animal oils such as corn oil, olive oil, soybean oil and fish oil,
(5) carbohydrates such as squalene and squalane,
(6) free fatty acids such as oleic acid and linoleic acid in a fluid form, and
(7) tocopherols such as dl-α -tocopheryl acetate.

Among the above-mentioned oils, tocopherols such as dl-α -tocopheryl acetate are preferably employed together with other oils listed above, if necessary.

In accordance with the present invention, the weight ratio of the drug : the compound of formula (I) : co-surfactant : surfactant : oil is in the range of 1 : 0.1∼50 : 1∼100 : 5∼100 : 1∼100, preferably, 1 : 0.5∼20: 2∼50 : 5∼80 : 2∼50.

In addition, the inventive composition may comprise pharmaceutically acceptable additives for an oral administration, e.g., aromatics, anti-oxidants and preservatives.

The inventive composition may be prepared by mixing and dissolving said components uniformly, and forming a micro-emulsion having a particle size of less than 1 µm in an aqueous medium.

The pharmaceutical composition of the present invention may be formulated into various pharmaceutical preparations, e.g., tablet, pill, powder, hard or soft capsule, granule, coated and liquid preparations, in accordance with any of the conventional procedures. For instance, a powder may be prepared by mixing a drug, a compound of formula (I), lactose and a conventional inert excipient and processing the mixture into a powder; a capsule, by adding suitable additives, e.g., a disintegrating agent and a lubricant to the powder and filling the resulting mixture into a hard or soft gelatin capsule; a tablet, by adding a suitable additives to the powder and tableting the resulting mixture. However, it is more preferable to formulate the inventive composition into preparations wherein the drug exists in a dissolved state, e.g., solution, emulsion and micro-emulsion preparations which provide a constant absorption rate. A micro-emulsion may be prepared by mixing the above-mentioned components (1) to (5) and stirring the mixture into a uniform emulsion. The resulting solution is then emulsified in an aqueous medium to obtain a micro-emulsion containing minute emulsified particles having a diameter of less than 1 µm. The micro-emulsion may be formulated into a capsule by filling it into a hard or soft gelatin capsule in accordance with a conventional procedure.

As described above, the inventive composition gives a remarkably high *in vivo* bioavailability of hardly absorbable drugs while showing little adverse effect.

The following Examples are intended to further illustrate the present invention without limiting its scope.

Further, percentages given below for solid in solid mixture, liquid in liquid, and solid in liquid are on a wt/wt, vol/vol and wt/vol basis, respectively, unless specifically indicated otherwise.

### Example 1: Preparation of Soft Capsule

A soft capsule was prepared using the following ingredients, wherein the compound of formula (I) is 1-(3-{[2-(3,4-dimethoxyphenyl)ethyl] methylamino}propyl)-4,5-dimethoxyindane- 1-carbonitrile:

| | Quantity(mg/capsule) |
|---|---|
| Paclitaxel | 10 |
| Dimethyl isosorbide | 400 |
| Cremophor® EL | 230 |
| Tween® 80 | 270 |
| D1-α -tocopheryl acetate | 250 |
| Erythorbic acid | 2 |
| Compound of formula (I) | 12 |

Paclitaxel, 1-(3-{[2-(3,4-dimethoxyphenyl)ethyl]methylamino}propyl)-4,5-dimethoxyindane-1-carbonitrile and dimethyl isosorbide, a co-surfactant, were mixed and dissolved uniformly, and other ingredients were added thereto and dissolved. Then, the resulting solution was filled into a soft gelatin capsule in accordance with the capsule preparation method described in Korea pharmacopoeia to obtain a soft gelatin capsule preparation.

### Example 2: Preparation of Soft Capsule

A soft capsule was prepared by the procedure of Example 1 except that 1-(3-{[2-(3,4-dimethoxyphenyl)ethyl]methylamino}propyl)-4,5-dimethoxyindane -1-carboxylic methyl ester was employed as the compound of formula (I).

### Example 3: Preparation of Soft Capsule

A soft capsule was prepared by the procedure of Example 1 except that 1-(3-{[2-(3,4-dimethoxyphenyl)ethyl]methylamino}propyl)-5,6-dimethoxy-1,2,3,4-tetrahydronaphthalene-1-carbonitrile was employed as the compound of formula (I).

### Example 4: Preparation of Soft Capsule

A soft capsule was prepared by the procedure of Example 1 except that 1-(3-{ [2-(2,3,4-trimethoxyphenyl)ethyl]methylamino}propyl)-5,6-dimethoxy-1,2,3,4-tetrahydronaphthalene-1-carbonitrile was employed as the compound of formula (I).

### Example 5: Preparation of Soft Capsule

A soft capsule was prepared using the following ingredients by the procedure of Example 1, wherein the compound of formula (I) is 1-(3-{[2-(3,4-dimethoxyphenyl)ethyl]methylamino }propyl)-4,5-dimethoxyindane-1-carbonitrile:

| | Quantity(mg/capsule) |
|---|---|
| Paclitaxel | 20 |
| Dimethyl isosorbide | 400 |
| Tween® 80 | 400 |
| Dl-α-tocopheryl acetate | 250 |
| Erythorbic acid | 2 |
| Compound of formula (I) | 20 |

### Example 6: Preparation of Soft Capsule

A soft capsule was prepared using the following ingredients by the procedure of Example 1 wherein the compound of formula (I) is 1-(3-{[2-(3,4-dimethoxyphenyl)ethyl]methylamino}propyl)-4,5-dimethoxyindane-1-carbonitrile:

| | Quantity(mg/capsule) |
|---|---|
| Paclitaxel | 30 |
| Dimethyl isosorbide | 400 |
| Ethanol | 130 |
| Tween® 80 | 430 |
| D1-α -tocopheryl acetate | 200 |
| Erythorbic acid | 2 |
| Compound of formula (I) | 20 |

### Example 7: Preparation of Soft Capsule

A soft capsule was prepared using the following ingredients by the procedure of Example 1 wherein the compound of formula (I) is 1-(3-{[2-(3,4-dimethoxyphenyl)ethyl]methylamino}propyl)-4,5-dimethoxyind ane-1-carbonitrile:

| | Quantity(mg/capsule) |
|---|---|
| Paclitaxel | 20 |
| Diethyleneglycol monoethylether | 365 |
| (Transcutol® ) | |
| Cremophor® EL | 180 |
| PEG6000-hydroxystearate | 300 |
| (Solutol® HS15) | |
| Medium chain triglyceride | 235 |
| (Miglyol® 812) | |
| Tween® 80 | 85 |
| Erythorbic acid | 2 |
| Compound of formula (I) | 20 |

### Example 8: Preparation of Soft Capsule

A soft capsule was prepared using the following ingredients by the procedure of Example 1 wherein the compound of formula (I) is 1-(3-{[2-(3,4-dimethoxyphenyl)ethyl]methylamino}propyl)-4,5-dimethoxyindane-1-carbonitrile:

| | Quantity(mg/capsule) |
|---|---|
| Cyclosporin A | 100 |
| Dimethyl isosorbide | 340 |
| HCO® -50 | 220 |
| Tween® 20 | 300 |
| Ethyl linoleate | 140 |
| Glyceryl monooleate | 120 |
| Compound of formula (I) | 50 |

### Example 9: Preparation of Tablet

A tablet was prepared using the following ingredients, wherein compound of formula (I) is 1-(3-{[2-(3,4-dimethoxyphenyl)ethyl]methylamino}propyl)-4,5-dimethoxyindane-1-carbonitrile:

| | Quantity(mg/capsule) |
|---|---|
| Paclitaxel | 30 |
| Lactose | 120 |
| Cremophor® EL | 10 |
| Microcrystalline cellulose | 70 |
| Sodium glycolate starch | 30 |
| Magnesium stearate | 2 |
| Compound of formula (I) | 30 |

Paclitaxel, 1-(3-{[2-(3,4-dimethoxyphenyl)ethyl]methylamino}propyl)-4,5-dimethoxyindane-1-carbonitrile, lactose and Cremophor EL were mixed uniformly and other ingredients were added thereto to obtain a mixture, which was then processed into a tablet having a degree of hardness of 5 by a method for preparing a tablet described in Korea Pharmacopoeia.

### Test Example 1: In Vivo Absorption Test of Paclitaxel

In order to investigate the bioavailability of paclitaxel contained in the inventive preparations, *in vivo* absorption tests were carried out as follows for the soft capsules of Examples 1 to 4 and, as a control, a soft capsule prepared by employing the ingredients of Example 1 except for the compound of formula (I).

Twenty-five 14- to 15-week-old male Sprague-Dawley rats each weighing about 300 g were fasted for over 48 hours while they were allowed free access to water, and then divided into five groups each containing 5 rats.

The five groups of rats were orally administered with the inventive preparations of Examples 1 to 4 and the control preparation-(A), respectively, in a dose of 20 mg paclitaxel/kg body weight of the rat. Blood samples were taken directly from the hearts of the rats before and 2, 4, 6, 8 and 24 hours after the administration.

The blood samples were centrifuged at 12,000 rpm to obtain serum samples therefrom. To 200 µℓ each of the serum samples was added 400 µℓ of acetonitrile as an internal standard and the mixture was shaken to obtain an extract. The extract was centrifuged at 1,000 rpm for 5 min. to obtain a supernatant. 50 µℓ of the supernatant was subjected to semi-micro HPLC under the following conditions:
- semi-micro HPLC system: Shiseido SI-1
- analysis column: Capcell Pak C₁₈ UG120(5 µm, 1.5 x 250 mm)
- pre-column: Capcell Pak MF Ph-1(4.6 x 10 mm)
- concentration column: Capcell Pak C₁₈ UG120(5 µm, 2.0 x 35mm)
- mobile phase for pre-column: 20 % acetonitrile
- mobile phase for analysis column: 55 % acetonitrile
- injection volume: 80 µℓ
- flow rate: 100 µℓ/min.
- detector: 227 nm

The time-dependent changes in blood paclitaxel concentrations of the rats are shown in Table 1 and Fig. 1:

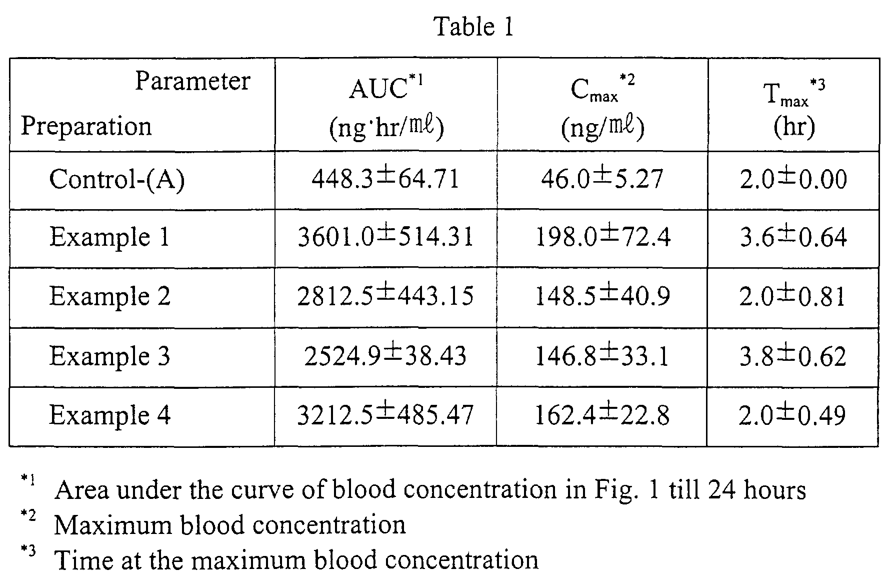

The results in Table 1 and Fig. 1 show that the bioavailabilities of paclitaxel observed for the inventive preparations are much higher as compared to the control preparation-(A) which does not contain the compound of formula (I).

### Test Example 2: In Vivo Distribution Test of Paclitaxel

In order to investigate the distribution of paclitaxel contained in the inventive preparation in the animal tissues, the drug concentrations in various rat tissues at 2.5 hours after the oral administration of the preparation were examined using the soft capsule of Example 1. As a control, a soft capsule was prepared by employing the ingredients of Example 1 except for the compound of formula (I).

Ten 14- to 15-week-old male Sprague-Dawley rats each weighing about 300 g were fasted for over 48 hours while they were allowed free access to water, and then divided into two groups each containing 5 rats.

The two groups of rats were orally administered with the inventive preparation of Example 1 and the control preparation, respectively, in a dose of 20 mg paclitaxel/kg body weight of the rat. 2.5 hours after the administration, blood samples were taken directly from the hearts of the rats and tissue samples were taken from the liver, lung, kidney and brain.

Blood paclitaxel concentration was measured by the procedure of Example 1. 1 g each of the tissue samples was washed with physiological saline and 2 mℓ of 0.5 M KH₂PO₄ solution(pH 4.5) was added thereto. The mixture was homogenized by centrifuging at 10,000 rpm for 10 min. and extracted three times with 5 mℓ of acetonitrile. The extracts were combined and centrifuged to obtain an organic phase. The organic phase was concentrated by evaporation under a nitrogen atmosphere at 30°C. The resulting concentrate was dissolved in 500 µℓ of 50 % acetonitrile and analyzed by the semi-micro HPLC of Test Example 1. The result is shown in Table 2.

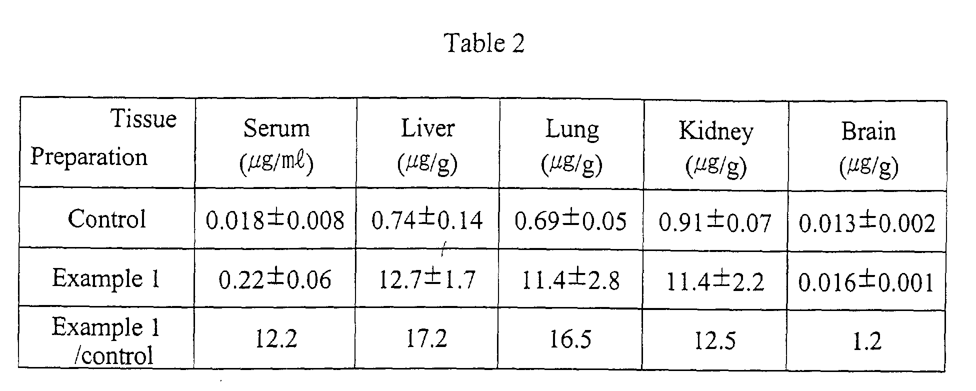

The result in Table 2 shows that the concentrations of paclitaxel in the blood, liver, lung and kidney observed at 2.5 hours after administering the inventive preparations are higher by a factor of more than 10 than those observed for the control.

### Test Example 3: In Vivo Absorption Test of Cyclosporin A

In order to investigate the bioavailability of cyclosporin A containd in the inventive preparation, in vivo absorption tests were carried out as follows for the soft capsule of Example 8 and, as a control, a soft capsule prepared by employing the ingredients of Example 8 except for the compound of formula (I).

Ten 14- to 15-week-old male Sprague-Dawley rats each weighing about 280 g were fasted for over 24 hours while they were allowed free access to water, and then divided into two groups each containing 5 rats.

The two groups of rats were orally administered with the inventive preparation of Example 8 and the control preparation-(B), respectively in a dose of 15 mg cyclosporin A/kg body weight of the rat. Blood samples were taken directly from the femoral vein before and 1, 2, 3, 4, 5.5, 8, 24, 48 hours after the administration.

To 10 ml capacity glass tube containing 1.0 ml each of the blood samples from the rats, 50µl of an internal standard solution (Cyclosporin D, 1000ng) and 500µl of a saturated sodium chloride solution were added and mixed for 2 minutes. The mixture was extracted with 2.0 ml of diethylether for 2 minutes and centrifuged at 3000 rpm for 10 minutes. The 1.5 ml of aliquot of the clear supernatant was washed with 3 ml of n-hexane and eluted with 3 ml of methanol through a solid-phase silicagel column (Bakerbond, 3 ml, J. T. Baker, USA) as an extraction column. Before use, the column was washed with 3 ml of dichloromethane followed by 3 ml of n-hexane. The eluent was evaporated to dryness under nitrogen stream and the residue was reconstituted with 200 µl of mobile phase, of which 100 µl was subjected to HPLC under the following conditions:
- HPLC system: Hitachi D7000 series
- analysis column: µ-Bondapak® C18 column (3.9 × 300 mm ID, 10µm, Waters, USA)
- mobile phase: acetonitrile:methanol:water = 55:15:35 (v/v)
- detector: UV 210 nm
- flow rate: 1.0 ml/min

The time-dependent changes in blood cyclosporin A concentrations of the rats are shown in Table 3 and Fig. 2:

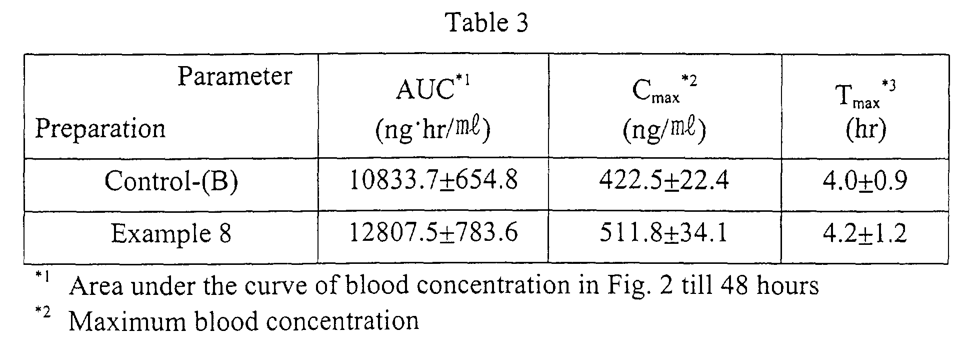

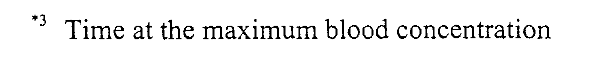

The results in Table 3 and Fig. 2 show that bioavailability of cyclosporin A observed for the inventive preparation is higher as compared to the control preparation-(B) which does not contain the compound of formula (I).

These results demonstrate that the inventive preparation is very useful for enhancing the bioavailability of a hardly absorbable drug with little adverse effect.

While the invention has been described with respect to the above specific embodiments, it should be recognized that various modifications and changes may be made to the invention by those skilled in the art which also fall within the scope of the invention as defined by the appended claims.

## Claims

1. An oral composition comprising a drug which by itself is not readily absorbed in the digestive tract and a compound of formula (I): 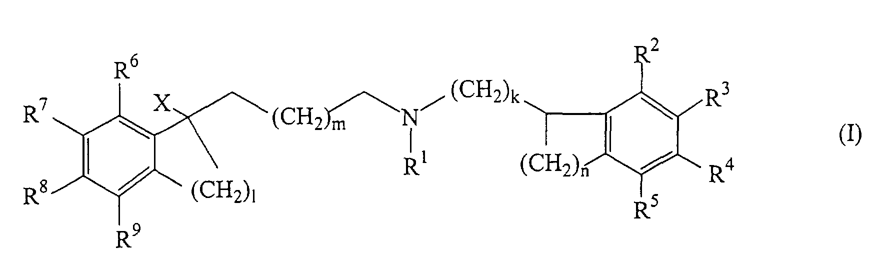 wherein,
X is CN, COOH, COOR¹⁰(wherein R¹⁰ is C₁₋₂ alkyl), SO₂Ph or SPh;
k, l, m and n are each independently an integer of 0 to 4;
R¹ is H or C₁₋₃ alkyl; and
R², R³, R⁴, R⁵, R⁶, R⁷, R⁸ and R⁹ are each independently H, OH or C₁₋₃ alkoxy.

2. The composition of claim 1, wherein the drug is selected from the group consisting of: actinomycin D, doxorubicin, daunomycin, vincristine, vinblastine, colchicine, paclitaxel, docetaxel, etoposide, hydroxyrubicin, cyclosporin A, FK-506, verapamil and nicardipine.

3. The composition of claim 1, wherein the drug is paclitaxel or cyclosporin A.

4. The composition of claim 1, wherein the compound of formula (I) is selected from the group consisting of: 1-(3-{[2-(3,4-dimethoxyphenyl)ethyl] methylamino}propyl)-4,5-dimethoxyindane-1-carbonitrile, 1-(3-{[2-(3,4-dimethoxyphenyl)ethyl]methylamino}propyl)-4,5-dimethoxyindane-1-carboxylic methyl ester, 1-(3-{[2-(3,4-dimethoxy phenyl)ethyl]methylamino} propyl)-5,6-dimethoxy-1,2,3,4-tetrahydronaphthalene-1-carbonitrile, and 1-(3-{[2-(2,3,4-trimethoxyphenyl)ethyl]methylamino}propyl)-5,6-dimethoxy-1,2,3, 4- tetrahydronaphthalene-1-carbonitrile.

5. The composition of claim 1 which comprises 0.1 to 50 parts by weight of the compound of formula (I) based on 1 part by weight of the drug.

6. The composition of claim 1 which further comprises a co-surfactant, a surfactant and an oil.

7. The composition of claim 6, wherein the weight ratio of the drug : the compound of formula (I) : co-surfactant : surfactant : oil is in the range of 1 : 0.1∼50 : 1∼100 : 5∼100 : 1∼100.

8. The composition of claim 6, wherein the co-surfactant is selected from the group consisting of: ethanol, propylene glycol, polyethylene glycol, propylene carbonate, transcutol, glycofurol, dimethyl isosorbide and a mixture thereof.

9. The composition of claim 6, wherein the surfactant is selected from the group consisting of: polyoxyethylene glycolated natural or hydrogenated vegetable oils, polyoxyethylene-sorbitan-fatty acid esters, polyoxyethylene fatty acid esters, polyoxyethylene-polyoxypropylene copolymer, polyoxyethylene-polyoxypropylene block copolymer, sodium dioctyl sulfosuccinate, sodium lauryl sulfate, phospholipids, propylene glycol mono- or di-fatty acid esters, trans-esterification products of natural vegetable oil triglycerides and polyalkylene polyols, monoglycerides, diglycerides, mono/di-glycerides, sorbitan fatty acid esters, sterols or their derivatives, and a mixture thereof.

10. The composition of claim 6, wherein the oil is selected from the group consisting of: fatty acid triglycerides, mono-, di- or mono/di-glycerides, esters of fatty acids and monovalent alkanols, natural vegetable or animal oils, squalene, squalane, oleic acid, linoleic acid, tocopherols, and a mixture thereof.
